# FASCICULE DE BREVET EUROPEEN

(11) **EP 2 462 963 B1**
(45) Date de publication et mention de la délivrance du brevet: **28.01.2015**
(21) Numéro de dépôt: 11191951.0
(22) Date de dépôt: 05.12.2011
(51) Int. Cl.: A61L 31/04, A61L 31/10

(54) **Procédé de fabrication d'un dispositif médical de renfort**
Herstellungsverfahren einer medizinischen Aussteifungsvorrichtung
Method for manufacturing a medical reinforcement device

(30) Priorité: 08.12.2010 FR 1060274
(43) Date de publication de la demande: 13.06.2012
(73) Titulaire: Les Laboratoires Brothier, 92000 Nanterre (FR)
(72) Inventeur: Barikosky, Michel, 75007 Paris (FR); Girardiere, Christian, 75116 Paris (FR)
(74) Mandataire: Bloch, Gérard

(56) Documents cités:
- WO-A1-2004/075942

## Description

La présente invention se rapporte à la fabrication d'un dispositif médical résorbable de renfort de zones tissulaires.

L'invention trouve particulièrement application dans le domaine des anastomoses mécaniques.

L'anastomose est un acte chirurgical manuel ou mécanique qui consiste à rétablir la connexion entre deux conduits anatomiques de même nature. Cet acte est fortement utilisé en chirurgie viscérale où le risque d'apparition d'effets indésirables postopératoires, tels des fistules et des fuites, reste important, notamment après une anastomose manuelle. L'utilisation ces dernières années de techniques mécaniques utilisant des pinces d'agrafage circulaires, a à la fois facilité le geste du chirurgien et la sécurité du patient en diminuant notamment l'apparition de fistules. Toutefois, le risque de complications comme des fuites ou une sténose et le taux de mortalité restent significatifs.

Afin de diminuer ces complications et ce taux de mortalité, de nouvelles stratégies ont été mises en place avec l'utilisation de dispositifs de renfort afin d'étanchéifier les agrafages, réduire les saignements et diminuer les tensions sur les tissus adjacents. Certains de ces dispositifs renforts sont résorbables mais d'autres ne le sont pas. Des exemples de dispositifs de renfort peuvent comprendre des dispositifs en PTFE (PolyTétraFluoroEthylène), des dispositifs de péricarde bovin, et sont constitués de polymères résorbables à base de cellulose ou de tissus intestinaux porcins.

Cependant, ces dispositifs présentent de nombreux inconvénients notamment un prix élevé, une innocuité virale incertaine ainsi que des risques dûs aux prions.

Le document FR 2 789 885 décrit un dispositif de renfort de suture comprenant un tissu en matériau à base d'alginate destiné à être emmanché sur une mâchoire d'une pince de suture. Pour fabriquer ce dispositif, on alimente en fibres d'alginate de calcium un métier à tricoter linéaire. On réalise un tricot tubulaire en jersey dont le diamètre est sensiblement égal à celui de la mâchoire de la pince. On enfile le tricot sur une forme dont le matériau est anti-adhésif. On plonge la forme habillée du tricot dans un bain contenant une solution aqueuse à 3% d'ester d'alginate afin d'en enduire le tricot. On élimine le surplus de solution, puis on sèche à l'air chaud, à 45°C selon l'exemple donné. Enfin on retire le dispositif. L'alginate, formant la nappe textile et le matériau d'enduction, présente des propriétés intéressantes pour les sutures. Il facilite la cicatrisation tissulaire. Il confère aux dispositifs une bonne résistance mécanique pour renforcer la plaie suturée durant la cicatrisation.

Au contact du sang, l'alginate d'enduction se ramollit et se charge de liquide biologique. Il se répand sur toute la surface du tissu et enveloppe les agrafes en obturant tous les interstices de la nappe et les perforations en assurant ainsi l'étanchéité à l'air et aux liquides. Le procédé décrit dans ce document ne prend pas suffisamment en compte les réactivités chimiques des matériaux en présence, c'est à dire l'alginate de calcium du support et la solution d'ester d'alginate de l'enduction.

En effet, il existe une incompatibilité de base entre ces deux espèces chimiques : une fibre d'alginate de calcium se gélifie, se désagrège et se solubilise progressivement dans une solution d'ester d'alginate jusqu'à disparaître. Même si on sèche rapidement le matériau après l'étape d'immersion, le produit réalisé est imparfait. Il apparaît notamment fragilisé mécaniquement.

En outre, le dispositif de renfort décrit n'est pas adapté pour être utilisé seul sans pince mécanique.

La présente invention vise à améliorer la situation en proposant un procédé de fabrication d'un dispositif de renfort résolvant tous ces problèmes.

A cet effet, la présente invention concerne un procédé de fabrication d'un dispositif médical résorbable de renfort de zones tissulaires, comprenant les étapes de :
- fabrication d'une gaine à partir d'alginate de calcium ;
- montage de la gaine sur une forme en matériau antiadhésif;
- enduction de la gaine au moyen d'un polyacide partiellement estérifié ou éthérifié (PAE) en solution, par exemple un ester d'alginate comme l'alginate de propylène glycol (PGA) ou l'alginate de butylène glycol ;
- découpage de la gaine enduite dans le sens de la longueur pour obtenir un tissu plat.

De manière remarquable, le découpage de la gaine étant effectué après l'étape d'enduction, tout risque de démaillage du tissu de la gaine, en périphérie ou au centre, en raison des forces de rétractation, est évité.

De façon optimale, le dispositif de renfort obtenu présente une face interne textile d'alginate de calcium ayant des capacités d'adhésion tissulaire in situ de sorte que cette face est dite pro-adhésiogène. Il présente de même une face externe plastique non adhésiogène et de porosité contrôlée, par exemple en PGA ou encore en alginate de butylène glycol.

Grâce au procédé de fabrication de l'invention, on obtient un dispositif médical biorésorbable permettant de diminuer les risques liés à l'utilisation de pinces mécaniques, lors d'une anastomose, grâce à un contrôle de l'hémostase et de la cicatrisation dans le temps des tissus reconnectés afin de renforcer l'anastomose par voie naturelle et éviter ainsi l'apparition de fistules ou de sténose.

Avantageusement, l'étape de fabrication de la gaine comprend une étape préalable de fabrication d'un fil d'alginate de calcium de type guluronique et une étape de réalisation d'un tricot avec le fil.

De préférence, le titre du fil d'alginate de calcium est choisi entre 100 et 500 dTex. Le rapport molaire entre les deux monomères de base : acide mannuronique M et acide guluronique G est choisi tel que M/G est inférieur à 1.

Selon une réalisation, le tricot est réalisé avec une tricoteuse circulaire. La maille du tricot est un jersey bloqué de type « locked stitches » avec une jauge 32. Ce maillage extrêmement serré permet une enduction efficace.

Avantageusement, le polyacide partiellement estérifié ou éthérifié avec lequel la gaine est enduite présente un taux d'estérification ou d'éthérification supérieur à 70% et inférieur à 90%, de préférence voisin de 80%.

Le lien entre la face textile d'alginate de calcium et la face plastique de PAE se fait par interaction électrostatique entre les ions calcium présents dans le textile et les fonctions carboxyliques libres du PAE. Il n'est donc pas nécessaire d'ajouter un agent de collage. En outre, un degré d'estérification ou d'éthérification trop faible impliquerait un échange ionique trop important entre l'alginate de calcium et le PAE résultant en une fragilisation de la base textile avec une création en parallèle d'inhomogénéités, dans la face plastique, responsables d'une perte de ses propriétés anti-adhésiogènes. A contrario, un taux trop important réduirait les points de cohésion entre les matériaux, menant à un délaminage in situ du dispositif de renfort.

Avantageusement, la forme en matériau anti-adhésif possède une fente sur sa surface, l'étape de découpage étant réalisée en suivant cette fente.

La forme est par exemple en PTFE (plus connu sous le nom de la marque protégée Téflon). Elle est de forme cylindrique de diamètre sensiblement identique à celui de la gaine, la fente étant prévue sur sa surface parallèlement à sa droite génératrice.

De préférence, la forme peut être mise en rotation autour de son axe.

Avantageusement, l'étape d'enduction comprend une pulvérisation de PAE et un séchage de la gaine concomitant ou alterné.

Une rotation associée à la pulvérisation et au séchage permet de maîtriser finement la structure et la masse du film vitrifié ainsi formé.

Il est ainsi possible de lier deux matériaux antagonistes, à savoir l'alginate de calcium et le PAE, sans liant supplémentaire. Des buses de pulvérisation et un système de séchage sont idéalement placés en opposition pour la mise en oeuvre de l'enduction.

De préférence, la concentration de polyacide partiellement estérifié ou éthérifié dans ladite solution est comprise entre 2 et 5% en fonction de la viscosité de la solution qui est idéalement inférieure à 1 Pa.s.

Avantageusement, la teneur en eau résiduelle de la gaine après l'étape d'enduction, comprenant la pulvérisation et le séchage, est inférieure à 15%.

Des modes de réalisation de l'invention vont maintenant être décrits de façon plus précise mais non limitative en regard des dessins annexés sur lesquels :
- la figure 1 est un organigramme illustrant les étapes successives du procédé de fabrication selon l'invention ;
- la figure 2 est une vue en perspective d'une forme en matériau antiadhésif pour la mise en oeuvre du procédé de fabrication selon un mode de réalisation de l'invention ;
- la figure 3 est une vue de face d'un système mettant en oeuvre l'étape d'enduction du procédé de fabrication selon un mode de réalisation de l'invention; et
- la figure 4 est une vue de derrière du système de la figure 3.

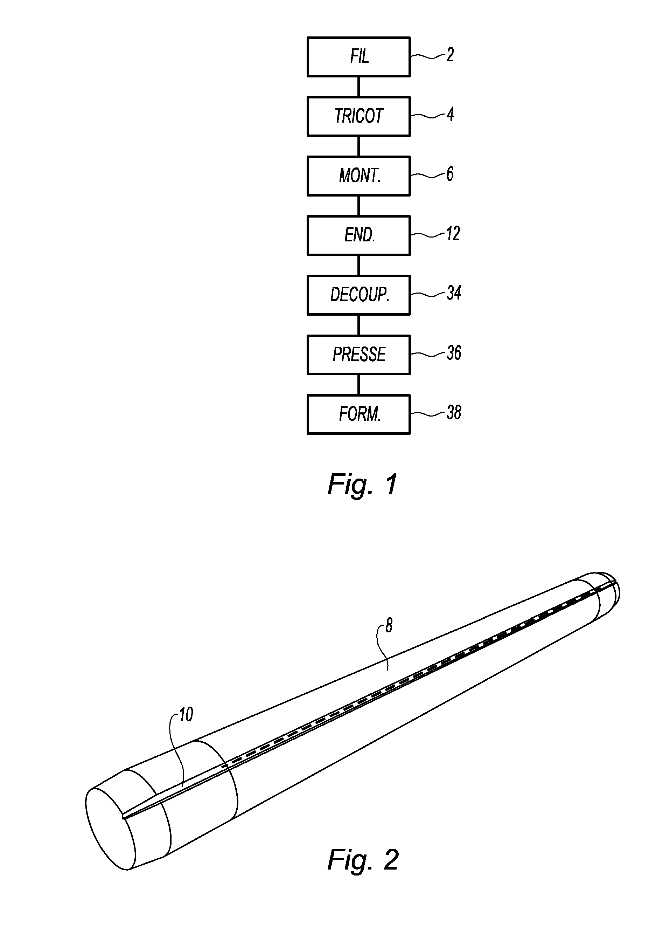

L'organigramme de la figure 1 illustre les étapes de fabrication d'un dispositif de renfort selon un mode préféré de réalisation de l'invention.

Lors d'une étape préalable 2, un fil d'alginate de calcium est fabriqué de manière conventionnelle. Le titre du fil d'alginate de calcium est choisi entre 100 et 500 dTex (décitex). De préférence, l'alginate est de type guluronique. Plus particulièrement, le rapport molaire entre les deux monomères de base : acide mannuronique M et acide guluronique G est choisi tel que M/G est inférieur à 1.

A l'étape 4, un tricot tubulaire, formant une gaine tricotée, est réalisé avec le fil d'alginate de calcium à l'aide d'une tricoteuse circulaire. La maille du tricot est un jersey bloqué de type « locked stitches » avec une jauge 32. De préférence, le tricot obtenu possède un diamètre inter-maille supérieur à 75 µm.

Le diamètre de la gaine est choisi en fonction des dimensions souhaitées du dispositif de renfort et du diamètre de la pince circulaire sur laquelle le dispositif de renfort est destiné à être positionné.

La gaine est ensuite montée, lors d'une étape 6, sur une forme tubulaire 8, représentée sur la figure 2, de préférence circulaire.

Selon une réalisation préférée, la forme 8 est revêtue d'une couche anti-adhérente en PTFE. Elle est pourvue d'une fente 10 sur sa surface, parallèlement à sa droite génératrice.

L'ensemble de la gaine et de la forme 8 subit alors, à l'étape 12, une enduction de polyacide partiellement estérifié ou éthérifié (PAE) en solution. Cette étape d'enduction comprend une pulvérisation de polyacide partiellement estérifié ou éthérifié et un séchage de la gaine concomitant ou alterné.

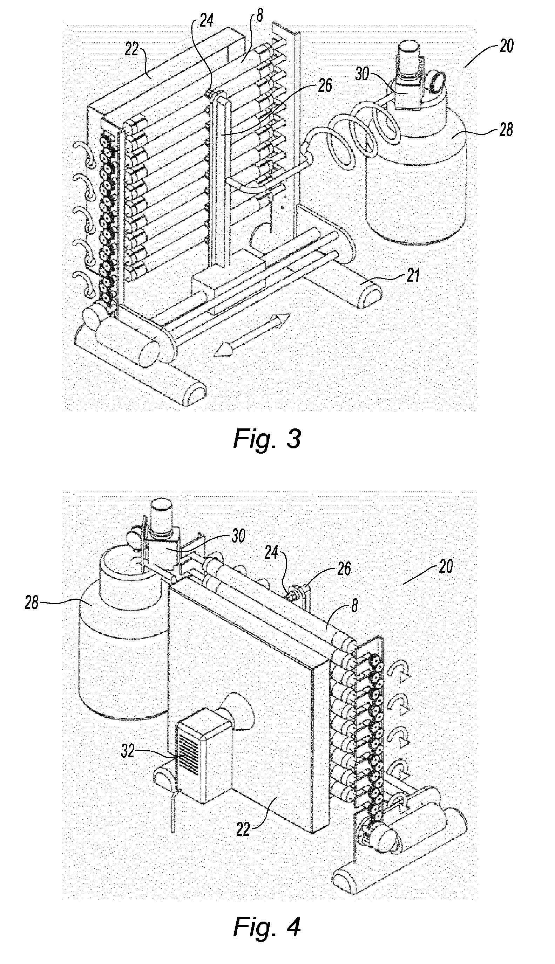

Les figures 3 et 4 illustrent un exemple de réalisation d'un système 20 mettant en oeuvre l'étape 12 d'enduction.

Le système 20 comprend un bâti 21 sur lequel sont montées horizontalement ici une pluralité de formes 8.

Le système 20 comprend en outre un ensemble de buses de pulvérisation 24 montées sur un support 26. Les buses 24 sont alimentées en solution de PAE par un pot 28 sous pression équipé d'un monodétenteur 30. En variante non représentée, le pot sous pression 28 peut être remplacé par une pompe à engrenage.

Ainsi que cela apparaît sur la figure 4, le système 20 comprend également un système de séchage 22, 32 placé en opposition par rapport aux buses de pulvérisation 24.

En fonctionnement, chaque forme 8 est animée d'un mouvement de rotation uniforme autour de son axe alors que le support 26 de buses se translate horizontalement dans un mouvement de va et vient, parallèlement à l'axe de la forme 8.

La forme 8 peut tourner autour de son axe en continu ou pas à pas à chaque translation du support 26 de buses.

L'épaisseur d'enduction de la gaine résultante est fonction notamment du temps de pulvérisation par unité de surface, du débit de pulvérisation et de la concentration de la solution de PAE pulvérisée. Cette concentration est comprise entre 2 et 5% en fonction de la viscosité de la solution qui est idéalement inférieure à 1 Pa.s.

Selon un autre mode de réalisation non représenté, la forme 8 et l'axe de translation du support 26 de buses sont verticaux. Le montage en horizontal est néanmoins préférable car il permet, en évitant d'avoir des gouttes de la solution lors de la pulvérisation, de maintenir une épaisseur d'enduction constante de la gaine et une homogénéité de la surface enduite.

Il est également possible de prévoir une seule buse 24 sur le support 26, la buse 24 étant alors animée d'un mouvement de translation pas à pas vertical et d'un mouvement continu de va et vient horizontal après chaque pulvérisation d'une forme.

Chaque pulvérisation de l'ensemble des formes est suivie ou assortie d'un séchage réalisé à l'aide du système de séchage 22,32 par convection d'air chaud afin d'amener la gaine à une teneur en eau résiduelle inférieure à 15% dans le produit fini.

On pourrait prévoir d'autres types de séchage, par exemple par rayonnement infrarouge, ou par contact, les formes étant chauffées par une résistance chauffante ou un fluide caloporteur.

Afin d'accélérer le séchage et limiter l'effet thermique sur la gaine, on peut prévoir de disposer les formes dans une enceinte sous vide avec un léger apport calorifique venant de l'extérieur ou de l'intérieur de l'enceinte.

Suite à l'étape d'enduction 12 mise en oeuvre par le système 20, une gaine enduite ayant une face interne d'alginate de calcium et une face externe de PAE est obtenue.

A titre d'exemples, qui ne doivent absolument pas être considérés comme limitatifs, la gaine obtenue présente les caractéristiques suivantes à l'analyse :
Exemple 1 :
   Grammage Tricot: 8-9 mg/cm²
   Grammage Enduction : 3-6 mg/cm²
   Sodium < 6%
   Alginate de Calcium > 94%
   Teneur en eau <15%.
Exemple 2 :
   Grammage Tricot: 4-4,5 mg/ cm²
   Grammage Enduction : 3-6 mg/ cm²
   Sodium < 6%
   Alginate de Calcium > 94%
   Teneur en eau <15%.
Exemple 3 :
   Grammage Tricot: 16-18 mg/ cm²
   Grammage Enduction : 3-6 mg/ cm²
   Sodium < 6%
   Alginate de Calcium > 94%
   Teneur en eau <15%.
Exemple 4 :
   Grammage Tricot : 8-9 mg/ cm²
   Grammage Enduction : 6-12 mg/ cm²
   Sodium < 6%
   Alginate de Calcium > 94%
   Teneur en eau <15%.

La gaine obtenue est alors découpée à l'étape 34 selon sa longueur, à l'aide d'une lame fine en suivant la fente 10 guide lame sur la forme 8. Ce découpage est représenté par des pointillés sur la figure 2.

Lors d'une étape 36, le tissu plan composite textile/plastique obtenu est mis sous presse pour conserver son aspect plan.

A l'étape 38, le tissu est découpé pour réaliser un dispositif de renfort ayant la forme souhaitée. Ce découpage est réalisé par un outil de découpe approprié, par exemple par écrasement, par cisaillement type poinçon-matrice, ou par d'autres techniques type jet fluide, etc.

Le dispositif médical de renfort réalisé grâce au procédé de fabrication selon l'invention a été testé lors d'anastomoses mécaniques colorectales sur porc et a montré son efficacité, notamment une accélération de la cicatrisation.

## Revendications

1. Procédé, de fabrication d'un dispositif médical résorbable de renfort de zones tissulaires, comprenant les étapes de :
- fabrication d'une gaine à partir d'alginate de calcium;
- montage (6) de la gaine sur une forme en matériau antiadhésif;
- enduction (12) de la gaine au moyen d'un polyacide partiellement estérifié ou éthérifié (PAE) en solution ;
- découpage (34) de la gaine enduite dans le sens de la longueur pour obtenir un tissu plat.

2. Procédé selon la revendication 1, dans lequel l'étape de fabrication de la gaine comprend une étape préalable de fabrication (2) d'un fil d'alginate de calcium de type guluronique et une étape de réalisation (4) d'un tricot avec le fil.

3. Procédé selon la revendication 1 ou 2, dans lequel le polyacide partiellement estérifié ou éthérifié avec lequel la gaine est enduite présente un taux d'estérification ou d'éthérification supérieur à 70% et inférieur à 90%, de préférence voisin de 80%.

4. Procédé selon l'une quelconque des revendications précédentes, dans lequel l'étape d'enduction (12) comprend une pulvérisation de polyacide partiellement estérifié ou éthérifié et un séchage concomitant de la gaine.

5. Procédé selon l'une quelconque des revendications 1 à 3, dans lequel l'étape d'enduction (12) comprend une pulvérisation de polyacide partiellement estérifié ou éthérifié et un séchage alterné de la gaine.

6. Procédé selon l'une quelconque des revendications précédentes, dans lequel la concentration de polyacide partiellement estérifié ou éthérifié dans ladite solution est comprise entre 2 et 5%.

7. Procédé selon la revendication 6, dans lequel la viscosité de la solution est inférieure à 1 Pa.s.

8. Procédé selon l'une quelconque des revendications précédentes, dans lequel la teneur en eau résiduelle de la gaine après l'étape d'enduction est inférieure à 15%.

## Patentansprüche

1. Verfahren zur Herstellung einer resorbierbaren medizinischen Einrichtung zur Verstärkung von Gewebszonen, umfassend die folgenden Schritte:
- Herstellung einer Hülle aus Kalziumalginat;
- Montage (6) der Hülle auf einer Form aus einem Antihaftmaterial;
- Bestreichen (12) der Hülle mit Hilfe einer teilweise esterifizierten oder ätherifizierten Polysäure (EPS) in Lösung;
- Ausschneiden (34) der bestrichenen Hülle in Richtung der Länge, um ein flaches Gewebe zu erhalten.

2. Verfahren nach Anspruch 1, bei dem der Schritt der Herstellung der Hülle einen vorherigen Schritt der Herstellung (2) eines Kalziumalginatfadens guluronischen Typs und einen Schritt der Herstellung (4) eines Gewebes mit dem Faden umfasst.

3. Verfahren nach Anspruch 1 oder 2, bei dem die teilweise esterifizierte oder ätherifizierte Polysäure, mit der die Hülle bestrichen wird, eine Esterifizierungs- oder Ätherifizierungsrate über 70 % und unter 90 %, vorzugsweise nahe 80 %, aufweist.

4. Verfahren nach einem der vorhergehenden Ansprüche, bei dem der Schritt des Bestreichens (12) eine Zerstäubung von teilweise esterifizierter oder ätherifizierter Polysäure und ein begleitendes Trocknen der Hülle umfasst.

5. Verfahren nach einem der Ansprüche 1 bis 3, bei dem der Schritt des Bestreichens (12) eine Zerstäubung von teilweise esterifizierter oder ätherifizierter Polysäure und ein alternierendes Trocknen der Hülle umfasst.

6. Verfahren nach einem der vorhergehenden Ansprüche, bei dem die Konzentration an teilweise esterifizierter oder ätherifizierter Polysäure in der Lösung zwischen 2 und 5 % beträgt.

7. Verfahren nach Anspruch 6, bei dem die Viskosität der Lösung geringer als 1 Pa.s ist.

8. Verfahren nach einem der vorhergehenden Ansprüche, bei dem der Gehalt an Restwasser der Hülle nach dem Schritt des Bestreichens unter 15 % % beträgt.

## Claims

1. Method for manufacturing an absorbable medical device for reinforcing tissue regions, comprising the steps of:
- manufacturing a sheath from calcium alginate;
- mounting (6) the sheath on a shape made of anti-adhesive material;
- coating (12) the sheath using a partially esterified or etherified polyacid (EPA) in solution;
- cutting (34) the coated sheath in the longitudinal direction to obtain a flat material.

2. Method according to claim 1, wherein the step of manufacturing the sheath comprises a prior step of manufacturing (2) a string of calcium alginate of the guluronic type and a step of producing (4) a knitted material using the string.

3. Method according to either claim 1 or claim 2, wherein the partially esterified or etherified polyacid with which the sheath is coated has an esterification or etherification rate of greater than 70 % and less than 90 %, preferably approximately 80 %.

4. Method according to any of the preceding claims, wherein the coating step (12) comprises spraying the sheath with partially esterified or etherified polyacid and simultaneously drying said sheath.

5. Method according to any of claims 1 to 3, wherein the coating step (12) comprises spraying the sheath with partially esterified or etherified polyacid and alternately drying said sheath.

6. Method according to any of the preceding claims, wherein the concentration of partially esterified or etherified polyacid in said solution is between 2 and 5 %.

7. Method according to claim 6, wherein the viscosity of the solution is less than 1 Pa·s.

8. Method according to any of the preceding claims, wherein the residual water content of the sheath after the coating step is less than 15 %.
